Europäisches Patentamt

European Patent Office

Office européen des brevets

(11) Publication number: **0 314 202**
**A1**

## (12) EUROPEAN PATENT APPLICATION

(21) Application number: **88120282.4**

(22) Date of filing: **20.02.86**

(51) Int. Cl.⁴: **C12N 15/00**

(30) Priority: **29.03.85 US 717319**

(43) Date of publication of application:
**03.05.89 Bulletin 89/18**

(60) Publication number of the earlier application in
accordance with Art.76 EPC: **0 196 762**

(84) Designated Contracting States:
**AT BE CH DE FR GB IT LI NL SE**

(71) Applicant: **CETUS CORPORATION**
**1400 Fifty-Third Street**
**Emeryville California 94608(US)**

(72) Inventor: **Horn, Glenn Thomas**
**3 Admiral Drive F370**
**Emeryville California 94608(US)**
Inventor: **Platak, Michael, Jr.**
**1120 Alfred Avenue**
**Walnut Creek California 94596(US)**

(74) Representative: **Bizley, Richard Edward et al**
**BOULT, WADE & TENNANT 27 Furnival Street**
**London EC4A 1PQ(GB)**

(54) **The isolation of mRNA.**

(57) An improved method for messenger RNA purification comprises either (a) treating an extract containing mRNA and ribosomal RNA with an oligo dT affinity column wherein the improvement comprises denaturing the exact prior to treating with said column or (b) treating the extract with an oligo dT affinity column and wherein the improvement comprises treating the extract with Sephadex G-100 before treating the extract with said column.

EP 0 314 202 A1

## THE ISOLATION OF mRNA

This invention relates to the purification or isolation of messenger RNA.

This invention provides improvements in the preparation of messenger RNAs which improvements are described below by way of illustration as applied to a process used in preparing the messenger encoding ricin B. Our copending European Patent Application No. 86301227.4, from which the present application is divided, is concerned with ricin toxin B fragment produced using recombinant means. The work described below is employed in the overall process described therein, but the present invention is not of course limited to such a process. The present specification incorporates by reference the entire contents of the said parent application.

One improvement of the present invention is specific for messenger RNA isolation from plant tissues, since plant extracts are accompanied by oxidized phenolics which are inhibitory to subsequent enzymatic manipulations such as, e.g., in vitro translation and reverse transcription. They can be removed by treating the preparation with Sephadex G-100. A further improvement, general to all mRNA preparations, comprises treating the preparation with a denaturant prior to the conventional dT affinity column separation of mRNA from ribosomal RNA.

In illustration of this invention, the mRNA encoding ricin B, and its associated proteins, was prepared using the method of Belamy, A.R., et al, Methods in Enzymology (1966) 104:156, with some modifications which effect improvements. These improvements and modifications overcome certain disadvantages of the standard methods. One such improvement succeeds in removing oxidized phenolic compounds from the preparations which constitute a problem in messenger RNA prepared from plant tissue sources. The other improvement has more general applicability in that it resolves a problem inherent in all mRNA preparation-- ie, the tendency of mRNA to associate with ribosomal RNA. If this association can be destroyed before application of the preparation to the conventional dT affinity column, the requirements for elution are much less.

In order to eliminate oxidized phenolics, the preparation is treated with Sephadex G-100. The gel retains the oxidized phenolics and passes the mRNA in the void volume. (Both polyphenolic compounds and transfer RNA are retarded.) This offers a simpler solution than that conventionally used, ie, prior isolation of the ribosomes or other subcellular component before carrying out RNA extraction.

To eliminate the ribosomal RNA complexing, the procedure is modified so as to react the suspension of RNA emerging from the foregoing G-100 column, if such column is used, with a denaturant, or in any event so reacting it prior to applying the preparation to a dT affinity column. The preparation can be denatured with a minimal amount of a conventional denaturing agent such as, for example, heat, formamide, or methyl mercuric hydroxide, preferably formamide. The denaturing agent breaks down the association between the ribosomal RNA and polyA portions of the messenger RNA and thus permits the messenger to adhere to the column more effectively.

The following Example is given to illustrate the invention.

### EXAMPLE

#### Isolation of mRNA from Castor Beans

50 g castor beans (Ricinus communis) were placed in 100 ml homogenizing buffer (150 mM NaCl, 50

*The vanadium ribonucleoside solution is prepared as follows: 893 mg $VOSO_4$ . $3H_2O$ was added to 2 ml water and the mixture boiled to dissolve the vanadium salt. A solution containing the 4 ribonucleosides was prepared by dissolving 1 mmole each of adenosine, cytidine, guanosine, and uridine in 17 ml water. Heat is required. 1 ml of the foregoing $VOSO_4$ solution was then added to the ribonucleoside solution and the resultant titrated to pH 6.5 with 10 N NaOH, and finally to pH 7 with 1 N NaOH while stirring in a boiling water bath. Formation of the complex is indicated by a change in color from bright blue to green-black. The solution was finally diluted to 20 ml with water.

mM Tris, pH 8.3, 5 mM EDTA and 50 mM freshly added β-mercaptoethanol) to which was added 12 ml 0.2 M vanadium-ribonucleoside complex, * 30 mg proteinase K, and 15 ml 20% SDS. The mixture was homogenized by blending at high speed for 3-4 min in a Waring blender and then incubating 2-3 hr at room temperature with occasional blending.

The suspension was centrifuged for 15 min at 8000 x g at 5° C and the pellet discarded. The supernatant was strained through cheesecloth to remove lipids and the filtrate extracted sufficiently with phenol:CHCl$_3$:isoamyl alcohol, 24:24:1 containing 1% hydroxy-quinoline, to remove vanadium salts and protein, and the aqueous layer brought to 0.4 M with NaCl and 10 mM with EDTA. 2.5 x volume absolute ethanol was added to precipitate nucleic acids, and the mixture stored at -20° C overnight.

The precipitate was centrifuged for 15 min at 7000 x g at 2° C, and the pellet resuspended in 9.5 ml aqueous solution 0.025 M NaCl, 0.025 M Tris, pH 8, plus 9.5 ml phosphate buffer (2.5 M total phosphate, K$_2$HPO$_4$:33% H$_3$PO$_4$, 20:1), plus 9.5 ml 2-methoxyethanol. The mixture was shaken and chilled on ice for 3-5 min with occasional mixing, and then centrifuged at 2000 x g for 5 min at 2° C. The upper layer was removed and to this was added 10 ml 0.2 M sodium acetate, and 5 ml 1% cetyl trimethylammonium bromide (CTAB) and the mixture chilled on ice for 10 min. The resulting white precipitate was harvested by centrifugation at 2000 x g for 10 min at 2° C. The precipitate was washed by addition of 70% ethanol in 0.1 M sodium acetate and by re-centrifuging at 2500 x g for 10 min at 4° C.

After removal of the supernatant, the pellet was resuspended in 2 ml G-100 column starting buffer (20 mM Tris, pH 8, 1 mM EDTA, 0.5% SDS), and then adjusted to contain 0.5 M NaCl. Solids were removed by centrifugation at 2000 xg for 5 min at room temperature and the supernatant applied to a Sephadex G-100 column (1.5 cm x 40 cm) and the column eluted using buffer similar to that applied to the column but lacking SDS. The eluate was assayed by monitoring OD$_{260}$. Desired messenger RNA was obtained in the flow through volume, leaving behind oxidized phenolic compounds present in the plant extract. (These compounds are known to behave similarly to polyA RNA on dT columns, inhibit protein synthesis, and thus interfere with the assay for mRNA.)

The initial peak containing mRNA was treated with formamide, a denaturant, to destroy ribosomal RNA complexing. To do this, the mRNA containing fractions were pooled, precipitated in ethanol, and the precipitates redissolved in a minimum volume of water. To this solution was added 9 volumes of deionized formamide containing 20 mM PIPES (piperazine-N,N-bis(2-ethanesulfonic acid), pH 6.5-7.0.

The mixture was then warmed to 37° C for 5 min, and 10 volumes of dT column buffer (0.5 M NaCl, 10 mM Tris, pH 7.5, 1 mM EDTA) added. The presence of formamide dissociates the polyA RNA from any ribosomal RNA present.

The denatured mixture was then run over an oligo dT column according to procedures well established in the art, and approximately 100 μg polyA RNA recovered upon elution.

As described in copending European Patent Application No. 86301227.4 the polyA mRNA prepared as in the preceding paragraph may be used to btain a cDNA library according to the method of Maniatis, et al Molecular Cloning, Cold Spring Harbor Laboratory (1982).

## Claims

1. An improved method for isolating mRNA which comprises treating an extract containing mRNA and ribosomal RNA with an oligo dT affinity column wherein the improvement comprises denaturing the exact prior to treating with said column.

2. An improved method of isolating mRNA from plant tissue which comprises treating the extract with an oligo dT affinity column and wherein the improvement comprises treating the extract with Sephadex G-100 before treating the extract with said column.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | METHODS IN MOLECULAR BIOLOGY, vol. 2, 1984, pages 117-120, New York, US; R.J. SLATER: "The purification of poly(A)-containing RNA by affinity chromatography" * Whole article * | 1,2 | C 12 N 15/00 |
| A | CHEMICAL ABSTRACTS, vol. 85, no. 17, 25th October 1976, page 245, abstract no. 118978h, Columbus, Ohio, US; L. GYENES et al.: "The use of affinity chromatography for the subfractionation of polyadenylated RNA on oligo (dT)-cellulose", & PROTIDES BIOL. FLUIDS, PROC. COLLOQ. 1975 (PUB. 1976). 23, 651-8 * Abstract * | 1,2 | |
| A | CHEMICAL ABSTRACTS, vol. 85, no. 5, 2nd August 1976, page 176, abstract no. 30221v, Columbus, Ohio, US; J.A. BANTLE et al.: "Specificity of oligo (dT)-cellulose chromatography in the isolation of polyadenylated RNA", & ANAL. BIOCHEM. 1976, 72(1-2), 413-27 * Abstract * | 1,2 | TECHNICAL FIELDS SEARCHED (Int. Cl.4)  C 12 N |

The present search report has been drawn up for all claims

| Place of search | Date of completion of the search | Examiner |
|---|---|---|
| THE HAGUE | 17-02-1989 | WIESER, M.R.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)